(19) Europäisches Patentamt
European Patent Office
Office européen des brevets

(11) EP 0 735 125 A2

(12) **EUROPÄISCHE PATENTANMELDUNG**

(43) Veröffentlichungstag: **02.10.1996 Patentblatt 1996/40**

(51) Int. Cl.[6]: **C09K 19/34**, C07C 33/02

(21) Anmeldenummer: **96104699.2**

(22) Anmeldetag: **25.03.1996**

(84) Benannte Vertragsstaaten: **CH DE FR GB LI NL**

(30) Priorität: **30.03.1995 DE 19511448**

(71) Anmelder: **BASF Aktiengesellschaft**
**67063 Ludwigshafen (DE)**

(72) Erfinder:
- **Siemensmeyer, Karl, Dr.**
  **67227 Frankenthal (DE)**
- **Tschierske, Carsten, Dr.**
  **06120 Halle (DE)**
- **Zab, Kerstin**
  **06110 Halle (DE)**

(54) **Flüssigkristalline Allene**

(57) Flüssigkristalline Allene der allgemeine Formel I

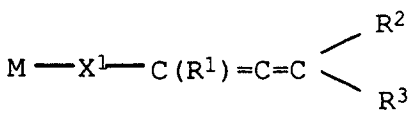

I

in der die Substituenten folgende Bedeutung haben:

| | |
|---|---|
| M | eine mesogene Gruppe |
| $R^1$, $R^2$ | Wasserstoff oder C-organische Reste mit 1 - 8 C-Atomen |
| $R^3$ | einen C-organischer Rest mit 1 - 30 C-Atomen |
| $X^1$ | $-(CH_2)_q-O-(CH_2)_p-$,<br>$-(CH_2)_q-NR^4-(CH_2)_p-$,<br>$-(CH_2)_q-CO-O-(CH_2)_p-$,<br>$-(CH_2)_q-O-CO-(CH_2)_p-$,<br>$-(CH_2)_q-CO-NR^4-(CH_2)_p-$<br>$-(CH_2)_q-NR^4-CO-(CH_2)_p-$ |
| $R^4$ | Wasserstoff oder einen Alkylrest mit 1-4 C-Atomen, |
| p | 1-20 und |
| q | 0-20, |

wobei die Definitionen für $R^2$ und $R^3$ auch vertauscht sein können und die Verwendung der Allenverbindungen in optischen Anzeigeelementen.

EP 0 735 125 A2

**Beschreibung**

Die vorliegende Erfindung betrifft neue flüssigkristalline Allene der allgemeine Formel I

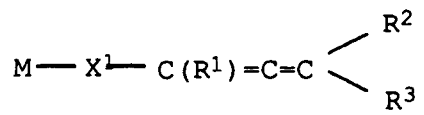

I

in der die Substituenten die folgende Bedeutung haben:

| | |
|---|---|
| M | eine mesogene Gruppe |
| $R^1$, $R^2$ | Wasserstoff oder C-organische Reste mit 1 - 8 C-Atomen |
| $R^3$ | einen C-organischen Rest mit 1 - 30 C-Atomen |
| $X^1$ | $-(CH_2)_q-O-(CH_2)_p-$,<br>$-(CH_2)_q-NR^4-(CH_2)_p-$,<br>$-(CH_2)_q-CO-O-(CH_2)_p-$,<br>$-(CH_2)_q-O-CO-(CH_2)_p-$,<br>$-(CH_2)_q-CO-NR^4-(CH_2)_p-$<br>$-(CH_2)_q-NR^4-CO-(CH_2)_p-$ |
| $R^4$ | Wasserstoff oder einen Alkylrest mit 1-4 C-Atomen, |
| p | 1-20 und |
| q | 0-20, |

wobei die Definitionen für $R^2$ und $R^3$ auch vertauscht sein können.

Weiterhin betrifft die Erfindung die Herstellung der neuen flüssigkristallinen Verbindungen, neue Allene als Zwischenprodukte zur Kopplung an mesogene Gruppen sowie die Verwendung der flüssigkristallinen Verbindungen in optischen Anzeigeelementen, in optischen, elektronischen sowie elektrooptischen Speichermedien, in elektrophotographischen Geräten sowie in lichtreflektierenden Schichten.

Flüssigkristalline Verbindungen werden bekanntermaßen auf dem elektrooptischen Gebiet für viele Zwecke eingesetzt. Zu nennen sind hier beispielsweise optische Speichersysteme (DE-A-38 27 603 und DE-A-39 17 196), die Elektrophotographie (DE-A-39 30 667), flüssigkristalline Anzeigeelemente wie Displays (Mol. Cryst. Liq. Cryst. 114, 151 (1990)) sowie bei ferroelektrischen flüssigkristallinen Medien elektrische Speichersysteme (Ferroelectrics, 104, 241 (1990)). Ferroelektrische Materialien zeigen eine permanente Polarisation, wenn sie in ein elektrisches Feld gebracht werden, d.h. sie behalten ihre Orientierung bei, nachdem das Feld wieder abgeschaltet wurde. Das Phänomen der Ferroelektrizität zeigen auch bestimmte flüssigkristalline Verbindungen, die eine chirale Struktur besitzen.

In der Schichtstruktur ferroelektrischer smektisch flüssigkristalliner C-Phasen (sog. $S_c$*-Phasen) sind die Moleküllängsachsen innerhalb der einzelnen Schicht gegenüber der Schichtnormalen Z geneigt. Die Richtung dieser Neigung wird durch den Direktor n angegeben. $S_c$*-Phasen weisen zwei stabile Zustände mit unterschiedlicher Richtung von n auf, zwischen denen durch Anlegen eines elektrischen Feldes geschaltet werden kann (elektrooptischer Effekt).

Ferroelektrische Flüssigkristalle finden vor allem in elektrooptische Anzeigeelementen Verwendung, die nach dem Prinzip des Surface Stabilized Ferroelectric Liquid Crystal (SSFCC) arbeiten. Diese Anzeigeelemente enthalten in der Regel Mischungen flüssigkristalliner Verbindungen, die entweder selbst chiral sind oder in denen durch die Anwesenheit chiraler Substanzen eine Chiralität induziert wird. An die Eigenschaften der flüssigkristallinen Verbindungen in diesem Anzeigeelementen werden hohe Anforderungen gestellt. Wünschenswert ist eine relativ niedrige Spontanpolarisation und eine geringe Rotationsviskosität, um hohe Schaltgeschwindigkeiten zu erreichen. Außerdem ist eine große Phasenbreite für die $S_c$*-Phase erwünscht. Hinsichtlich dieser Eigenschaften lassen die bekannten flüssigkristallinen Verbindungen zu wünschen übrig.

Der Erfindung lag daher die Aufgabe zugrunde, neue ferroelektrische Flüssigkristalle mit breiten enantiotropen flüssigkristallinen Phasen zu finden, welche eine geringe spontane Polarisation induzieren und gleichzeitig eine niedrige Rotationsviskosität aufweisen.

Demgemäß wurden die eingangs erwähnten flüssigkristallinen Allene gefunden.

Durch die Allengruppe wird eine chirale Gruppe in die mesogene Verbindung eingebracht, wodurch die besonders gewünschten ferroelektrisch flüssigkristallinen Eigenschaften auftreten können. Besonders bevorzugt sind daher solche erfindungsgemäßen Verbindungen, die nur ein Enantiomeres enthalten oder zumindest eine Mischung der beiden Enantiomeren, in denen das Enantionmerenverhältnis größer als 60:40 ist.

Die Substituenten $R^1$ und $R^2$ können Wasserstoff oder C-organische Reste mit 1-8 C-Atomen bedeuten. Neben reinen Alkylgruppen kommen auch durch Sauerstoff oder Stickstoff unterbrochene Ketten in Betracht, z.B. $-CH_2-CH_2-O-CH_2-CH_3$ oder $CH_2-CH_2-NH-CH_2-CH_3$. Bevorzugt sind jedoch kurze Alkylreste wie Methyl, Ethyl oder Propyl sowie Wasserstoff.

$R^3$ und $R^5$ bedeuten C-organische Reste mit 1 - 30 C-Atomen. Bevorzugt sind solche Reste, die eine im wesentlichen gestreckte Struktur aufweisen, also beispielsweise geradkettiges Alkyl oder durch Sauerstoff, Stickstoff, Ester- oder Amidgruppen unterbrochene Alkylketten. Bevorzugt sind Alkylreste mit 4 - 12 Kohlenstoffatomen, besonders bevorzugt solche mit 5 - 9 Kohlenstoffatomen, darunter ganz besonders der n-Heptylrest.

Zur Herstellung der flüssigkristallinen Verbindungen I geht man von Allenen der Formel II

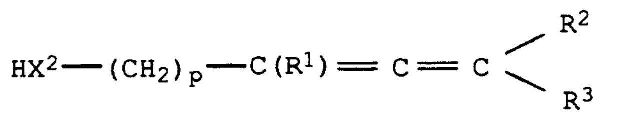

II

aus. Diese Allene II tragen zur Kopplung an die mesogene Gruppe M eine Hydroxyl-, Amino- oder Carboxylgruppe, welche über eine $-(CH_2)_p$-Gruppe mit der Allengruppe verbunden ist, wobei p eine Zahl von 1 - 20, bevorzugt 1 - 8, besonders bevorzugt 1 bedeutet. Besonders bevorzugt ist das Allenzwischenprodukt

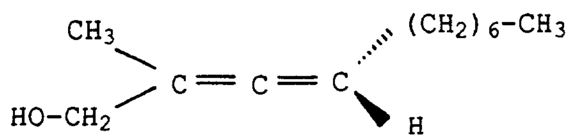

sowie die hierzu enantiomere Verbindung.

Als Zwischenprodukte zur Herstellung der flüssigkristallinen Allene der Formel I haben Allene der Formel IIa besondere Bedeutung

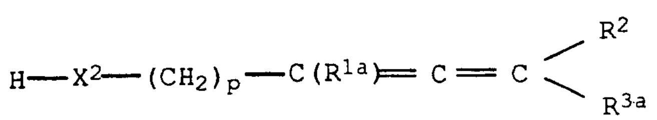

IIa

Bevorzugte Reste $R^{3a}$ sind diejenigen, wie sie für $R^3$ genannt wurden, die jedoch mehr als 6 C-Atome haben. Bevorzugt sind lineare Alkylgruppen mit 7 bis 11 C-Atomen, besonders bevorzugt der n-Heptylrest.

Da chirale Allene besondere flüssigkristalline Eigenschaften zeigen, sind solche auch für diese Zwischensprodukte besonders bevorzugt. Ebenfalls besonders bevorzugt sind Mischungen von Enantiomeren, in denen das eine Enantiomere in einem Überschuß von mindestens 60:40 vorliegt.

Die mesogene Gruppe M hat bevorzugt die Struktur Ia

$$R^5\text{-}X^{1a}\text{-}Z\text{-}A^1\text{-}Z\text{-}A^2\text{-}Z\text{-}(A^3)_m\text{-}Z\text{-}(A^4)_n\text{-} \qquad \text{Ia}$$

Sie hat eine im wesentlichen gestreckte Struktur und besteht bevorzugt aus 2 bis 4 ringförmigen Gruppen A, die über Brückenglieder Z miteinander verbunden sind. Als ringförmige Gruppen A kommen beispielsweise substituierte oder unsubstituierte iso- oder heteroaromatische Gruppen wie

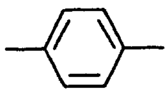 , 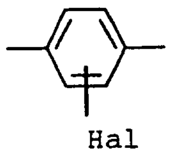 , 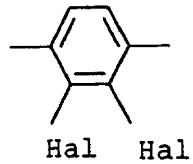 , 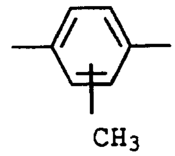 ,

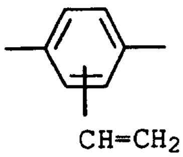 , 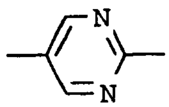 , 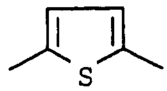 , 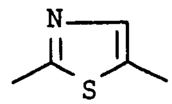 ,

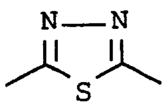 ,

wobi Hal Halogen oder Pseudohalogen, besonders bevorzugt F, Cl oder CN bedeutet, oder nichtaromatische Ringstrukturen wie

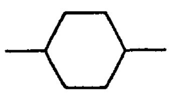 , 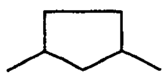 , 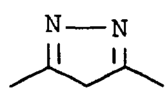 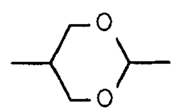

in Betracht.

Beispiele für besonders bevorzugte Molekülteile

$$-X^{1a}-A^1-Z-A^2-Z-(A^3)_m-Z-(A^4)_m-$$

sind

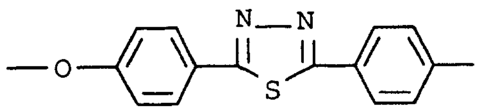

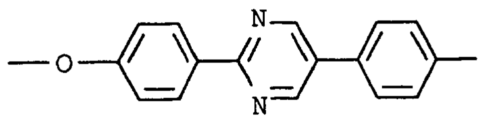

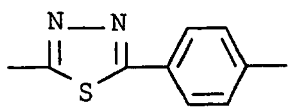

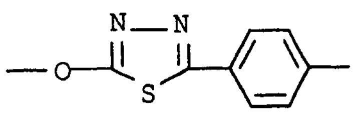

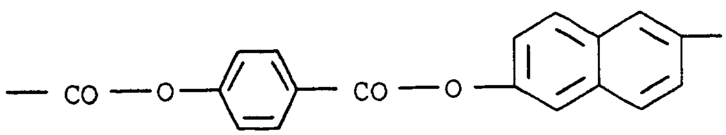

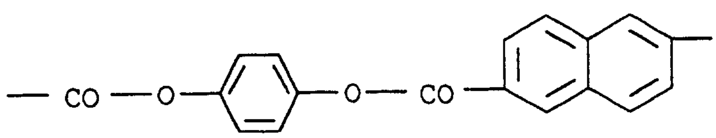

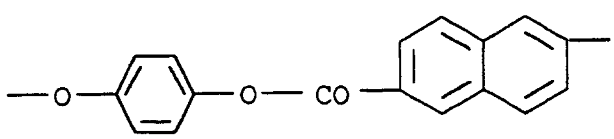

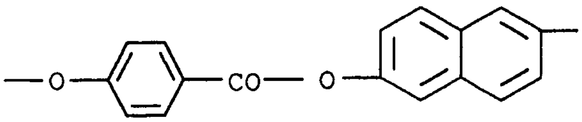

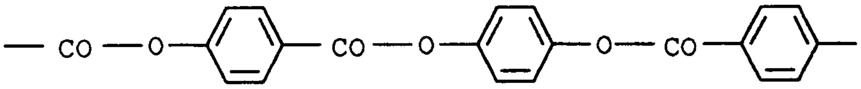

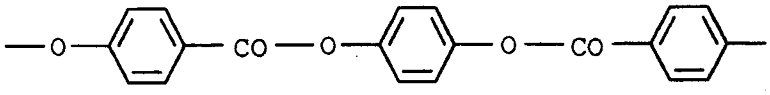

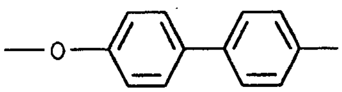

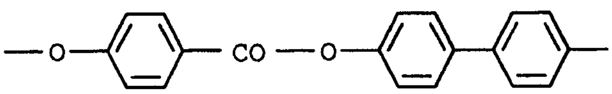

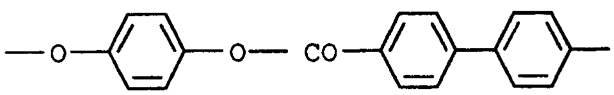

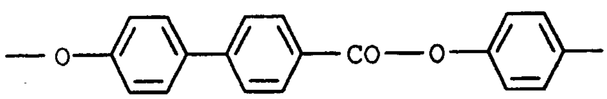

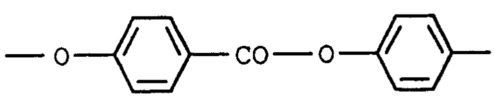

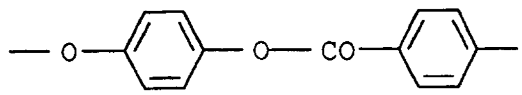

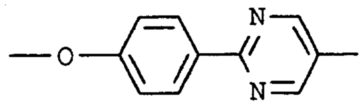

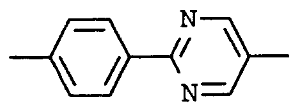

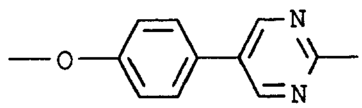

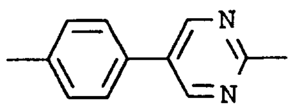

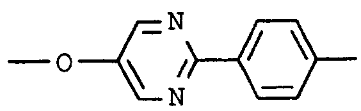

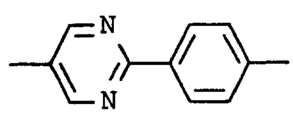

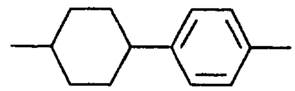

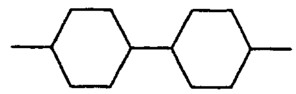

Die Herstellung der flüssigkristallinen Allene erfolgt vorzugsweise derart, daß die mesogene Gruppe und der Allenteil getrennt aufgebaut werden. Als funktionelle Kopplungsgruppe trägt der Allenteil eine Hydroxy-, eine Amino- oder eine Carboxylgruppe, wobei die Kopplung über eine Hydroxygruppe unter Ausbildung einer Etherbindung zur mesogenen Gruppe bevorzugt ist.

Die Allene II sind in an sich bekannter Weise über mehrere Stufen, beispielsweise in folgender Sequenz aus den entsprechenden Alkinolen erhältlich (für $X^2$ = $-NR^4-$ oder -O-CO- geht man von den entsprechenden Alkinaminen oder Alkincarbonsäuren aus).

a) Schutz des Alkinols (s. z.B. J. Org. Chem., 1982, 47, 2549)

$$HX^2\text{-}(CH_2)_p\text{-}C{\equiv}CH \xrightarrow{R^*Cl} R^*X^2\text{-}(CH_2)_p\text{-}C{\equiv}CH$$

R* ist eine Schutzgruppe, bevorzugt eine Silylgruppe, besonders bevorzugt tert.-Butyldiphenylsilyl.

b) Umsetzung des Alkins mit einer Carbonylverbindung (s. z.B. J. Org. Chem., 1975, 40, 2250)

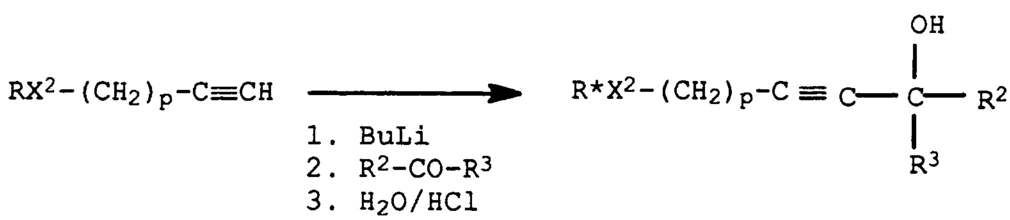


Zur Herstellung optisch aktiver Allene können die racemischen tertiären Alkohole entweder nach bekannten Methoden in die Enantiomeren getrennt werden und dann wie unter e) beschrieben bromiert werden oder, für den Fall, daß als Carbonylverbindung ein Aldehyd eingesetzt wurde, wie folgt umgesetzt werden:

c) Oxidation des Alkohols zum Keton (s. z.B. Tetrahedron Lett., 1975, 31, 2647)

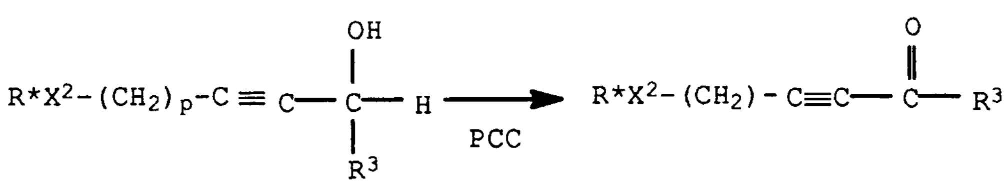


Die Oxidation kann mit verschiedenen Oxidationsmitteln druchgeführt werden, bevorzugt wird Pyridiniumchlorochromat (PCC) eingesetzt

d) Reduktion des Ketons, vorzugsweise mit einem optisch aktivem Reduktionsmittel (s. z.B. Tetrahedron, 1984, 40, 1371)

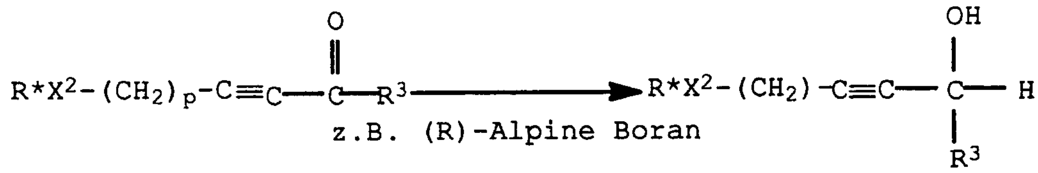


Als Reduktionsmittel werden bevorzugt optisch aktive Borane, besonders bevorzugt optisch aktives B-3-pinanyl-9-borabicyclo[3.3.1]-nonan (Alpine-Boran) eingesetzt.

e) Bromierung des Alkohols (s. z.B.: J.Org.Chem., 1991, 56, 1083)

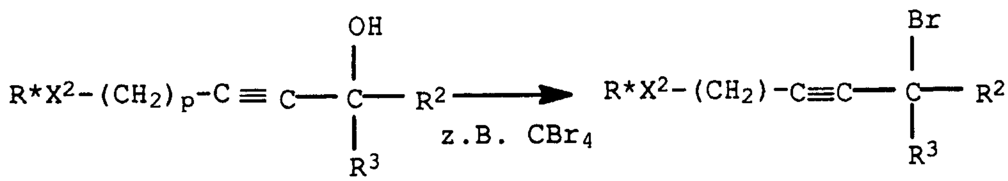


Als Bromierungsmittel wird bevorzugt $CBr_4$ in Gegenwart von Triphenylphosphin eingesetzt. Der Rest $R^2$ bedeutet hier Wasserstoff für den Fall, daß in Reaktionsschritt b) ein Aldehyd eingesetzt wurde. Wurde ein Keton eingesetzt, hat $R^2$ die Bedeutung eines C-organischen Rests mit 1-8 C-Atomen.

f) Umwandlung des Alkinbromids in ein substituiertes Allen (s. z.B.: J. Org. Chem., 1978, 43, 1389)

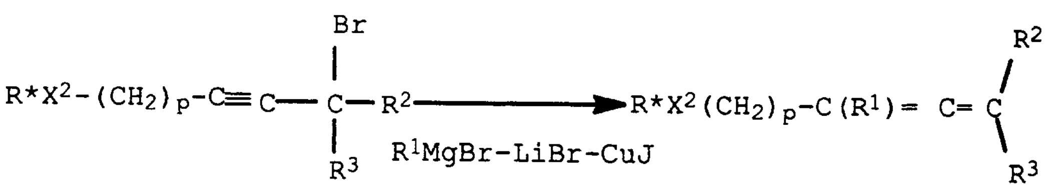


Die Allenbildung wird bevorzugt mit Hilfe eines Kuprats, welches z.B. in situ aus CuJ und einer Grignardverbindung gebildet wird, bewirkt. Durch diese Reaktion besteht die Möglichkeit, einen gewünschten Substituenten $R^1$ in das Allen einzuführen.

g) Abspaltung der Schutzgruppe (s. z.B. J. Org. Chem., 1991, 56, 1083)

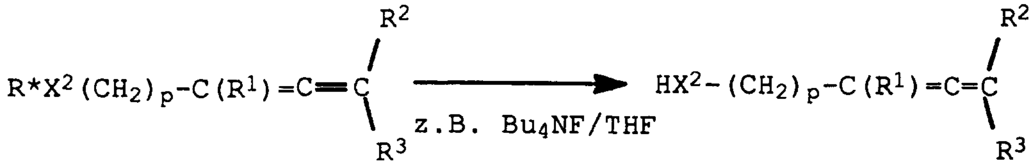


Die Abspaltung der Schutzgruppe wird im Falle einer Silylschutzgruppe bevorzugt mit Hilfe von Tetrabutylammoniumfluorid durchgeführt.

h) Kopplung des Allens mit einer mesogenen Verbindung

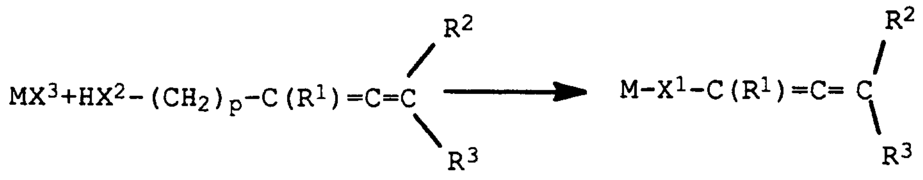

Das geeignete Kondensationsmittel für die Kopplung des Allens an die mesogene Gruppe hängt von der Natur der für die Kopplung vorgesehenen funktionellen Gruppen ab.

Ein Alkohol wird mit einer phenolischen Hydroxygruppe an der mesogenen Gruppe vorzugsweise mittels Diethylazodicarboxylat/Triphenylphosphin umgesetzt. Für die Kopplung einer Hydroxy- oder Aminogruppe mit einer Carboxylgruppe können beispielsweise Carbodiimide oder Carbonyldiimidazol verwendet werden.

Die erfindungsgemäßen flüssigkristallinen Allene können für sich oder in Kombination mit anderen Verbindungen verwendet werden. Dabei sind besonders Kombinationen mit solchen Substanzen vorteilhaft, die den Temperaturbereich oder die Breite der flüssigkristallinen Phasen in gewünschter Weise beeinflussen.

Flüssigkristalline Allene finden Verwendung bei der Herstellung von elektrooptischen Anzeigeelementen wie Displays, in optischen, elektronischen sowie elektrooptischen Speichermedien, in elektrophotographischen Geräten sowie in lichtreflektierenden Schichten. Besonders bevorzugt ist die Verwendung der erfindungsgemäßen Verbindungen in SSFLC-Displays, da sie durch ihre ferroelektrischen Eigenschaften, ihre relativ niedrige Spontanpolarisation und niedrige Rotationsviskosität ein besonders günstiges Schaltverhalten aufweisen.

Beispiele

Beispiel 1

Herstellung von (S)-2-Methyl-1{4-[5-(4-octyloxyphenyl)-1,3,4-thiadiazol-2-yl]phenoxy}undeca-2,3-dien[9a]

9a

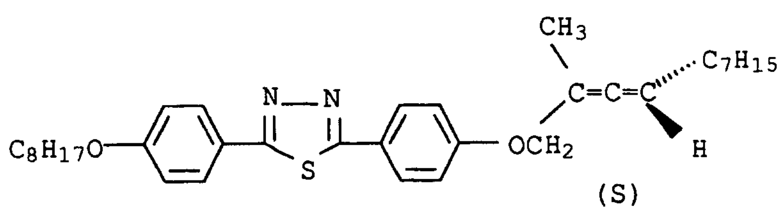

a) Herstellung von rac-1(tert.-Butyldiphenylsilyloxy)undec-2-in-4-ol[2]

15,9 g (54 mmol) des silylgeschützten Propargylalkoholes in 80 ml abs. THF wurden auf -78°C gekühlt und unter Argon mit 33 ml (3,5 g, 54 mmol) n-Butyllithium (1,6 M in Hexan) versetzt. Nach 30 min wurden 6,1 g (49 mmol) Octanal in 20 ml abs. THF zugetropft. Nach weiteren 3 h wurde bei 0°C mit 80 ml 10 gew.-%iger Salzsäure hydrolysiert. Die Aufarbeitung erfolgte durch Etherextraktion und Säulenchromatographie.

Ausbeute: 65 %

b) Herstellung von 1-(tert-Butyldiphenylsilyloxy)undec-2-in-4-on[3]

13 g [2] (30 mmol) in 30 ml abs. Dichlormethan wurden mit einer Suspension von 12,9 g (60 mmol) Pyridiniumchlorochromat in 60 ml abs. Dichlormethan bei Raumtemperatur vermischt. Nach 4 h Rühren wurden dem Gemisch 300 ml n-Pentan zugegeben. Die Lösung wurde dekantiert, der Feststoff mit Pentan gewaschen und die vereinigten organischen Extrakte filtriert und konzentriert. Der Rückstand wurde säulenchromatographisch gereinigt.

Ausbeute: 70 %

c) Herstellung von (R)-1-(tert-Butyldiphenylsilyloxy)undec-2-in-4-on[4]
3 g (7,1 mmol) [3] wurden mit 28,6 ml (14,2 mmol) einer 0,5 molaren (R)-Alpine-Boranlösung in THF bei 0°C unter Argon vermischt und dann 4 Tage bei Raumtemperatur gerührt. Anschließend wurden 0,56 ml (10 mmol) frisch destillierter Acetaldehyd zugetropft und eine weitere Stunde gerührt. Anschließend wurde das freigesetzte $\alpha$-Pinen im Vakuum abdestilliert. Dann wurden 5 ml abs THF, 5 ml 3 M NaOH und 5 ml 30 gew.-%ige $H_2O_2$-Lösung zugegeben, und es wurde 3 h bei 40°C gerührt. Die Aufarbeitung erfolgte durch Etherextraktion und Säulenchromatographie.
Ausbeute: 63 %
Enantiomerenreinheit: 88 % e.e.

d) Herstellung von (S)-4-Brom-1-(tert-Butyldiphenylsilyloxy)undec-2-in [5]
3,6 g (8,5 mmol) [4], 0,77 g (9,7 mmol) abs Pyridin und 5,4 g (20 mmol) Triphenylphosphin wurden in 50 ml abs THF gelöst und mit einer Lösung von 3,2 g (9,7 mmol) Tetrabromkohlenstoff und 0,1 ml abs Pyridin in 20 ml abs THF bei 20°C unter Rühren vermischt. Das Gemisch wurde 2 h unter Argon gerührt und dann mit 150 ml Hexan versetzt. Der ausgefallene Feststoff wurde abfiltriert, und die Lösung wurde zweimal nacheinander mit 10 %iger Salzsäure, gesättigter $NaHCO_3$-Lösung und gesättigter NaCl-Lösung gewaschen. Anschließend wurde mit $Na_2SO_4$ getrocknet, und das Lösungsmittel wurde unter vermindertem Druck abdestilliert. Das Rohprodukt wurde säulenchromatographisch gereinigt.
Ausbeute: 63 %

e) Herstellung von (S)-1(tert.-Butyldiphenylsilyloxy)-2-Methylundeca-2,3-dien [6]
Unter Argonatmosphäre wurden bei 0°C zu einer kräftig gerührten Suspension aus 2,1 g (24 mmol) Lithiumbromid und 4,6 g (24 mmol) Kupfer(I)jodid in 240 ml abs. THF 8 ml (24 mmol, 3 M in Ether) Methylmagnesiumbromid getropft. Das Reaktionsgemisch wurde bei 0°C 15 min gerührt und dann mit 3,9 g (8 mmol) [5], gelöst in abs. THF, versetzt. Nach einer weiteren Stunde bei 0°C wurden 50 ml gesättigte $NH_4Cl$-Lösung hinzugefügt, der ausfallende Feststoff abfiltriert und die organische Phase unter Vakuum vom Lösungsmittel befreit. Die Aufarbeitung erfolgte durch Etherextraktion und Säulenchromatographie.
Ausbeute: 80%

f) Herstellung von (S)-2-Methylundeca-2,3-dien-1-ol [7]
Zu einer Lösung von 2,3 g (5,5 mmol) [6] in 10 ml abs THF wurde unter Argon 16,5 ml (16,5 mmol) Tetra-n-butylammoniumfluorid (1 M in THF) zugetropft und das Gemisch 12 h gerührt. Anschließend wurde die Lösung konzentriert und durch Dichlormethanextraktion und Säulenchromatographie aufgearbeitet.
Ausbeute: 70 %
Enantiomerenreinheit: 52 % e.e.

g) Herstellung von (S)-2-Methyl-1-{4-[5-(4-octyloxyphenyl)-1,3,4-thiadiazol-2-yl]phenoxy}undeca-2,3-dien [9a]
o,2 g (1 mmol) [7], 0,26 g (0,67 mmol) 4-[5-(4-Octyl-oxyphenyl)-1,3,4-thiadiazol-2-yl]phenol [8a] und 0,26 g (1 mmol) trockenes Triphenylphosphin wurden in 10 ml abs. THF gelöst und unter Eiskühlung und Rühren mit einer Lösung von 0,17 g (1 mmol) Diethyl-azodiacarboxylat in 10 ml abs. THF versetzt. Es wurde 20 h bei Raumtemperatur gerührt und anschließend wurde das Lösungsmittel unter vermindertem Druck abgezogen. Die Reinigung erfolgte durch Umkristallisation aus Methanol-Wasser (10:1) und anschließend aus Methanol-Chloroform (3:1).
Ausbeute: 28 %

Beispiel 2

Herstellung von racemischem 9a

Die racemische Verbindung wurde durch direkte Umsetzung des geschützten Propargylalkohols [2] gemäß Beispiel 1d zum Bromid [rac-5] und analogen weiteren Umsetzungen erhalten.

Beispiel 3:

Herstellung von

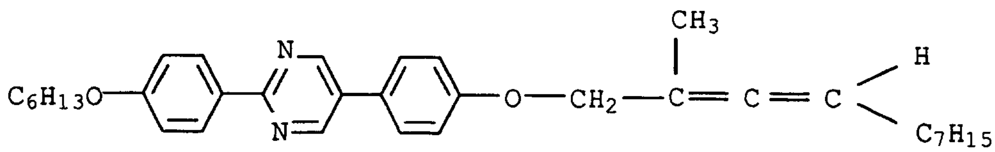

rac-9b

Die Verbindung wurde durch Kondensation des Allenalkohols 2-Methylundeca-2,3,-dien-1-ol (rac-7, analog zu Beispiel 1f erhältlich) mit der mesogenen Verbindung

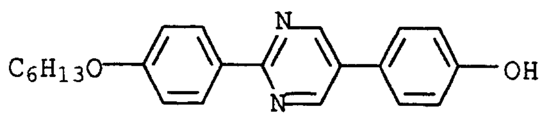

analog zur Umsetzung gemäß Beispiel 1g erhalten.

Beispiel 4

Es wurde das Phasenverhalten der in den Beispielen 1 - 3 beschrieben Verbindungen untersucht. Für flüssigkristalline Verbindungen insbesondere beim Einsatz in optischen Anzeigeelementen ist ein breiter Temperaturbereich der gewünschten flüssigkristallinen Phae, insbesondere der $S_c$- oder $S_c$*-Phase wünschenswert. In den vorliegenden Fällen wurden günstige $S_c$- bzw. $S_c$*-Phasenbreiten von ca. 30-40°C gezeigt.

Die Phasenumwandlungstemperaturen wurden wie üblich mit einem Polarisationsmikroskop (Leitz Ortholux II pol) in Verbindung mit einem Mikroskopheiztisch (Mettler FP 800/82) bestimmt.

Die Abkürzungen haben folgende Bedeutung:

| | | |
|---|---|---|
| cr | = kristallin | |
| $S_c$ | = smektisch flüssigkristallin | flüssig-kristalline Phasen |
| $S_c$* | = ferroelektrisch smektisch flüssigkristallin | flüssig-kristalline Phasen |
| N | = nematisch | flüssig-kristalline Phasen |
| N* | = ferroelektrisch nematisch | flüssig-kristalline Phasen |
| BP | = Blue Phase | flüssig-kristalline Phasen |
| is | = isotrop | |

Folgende Phasenumwandlungstemperaturen wurden für die Verbindungen nach den Beispielen 1 bis 3 gemessen:

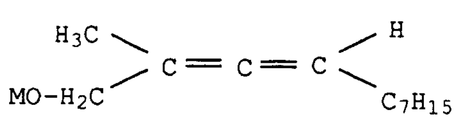

| | M | Phase | Temperaturbereich [°C] |
|---|---|---|---|
| (S)-9a (s.Bsp.1) | 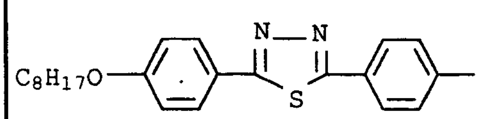 | Cr<br>$S_C^*$<br>N *<br>BP<br>is | < 67<br>67 - 99<br>99 - 101<br>101 - 102<br>> 102 |
| rac-9a (s.Bsp.2) | 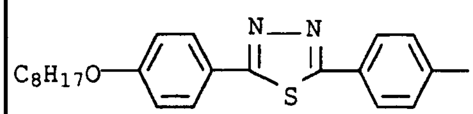 | Cr<br>$S_C$<br>N<br>is | < 64<br>64 - 96<br>96 - 99<br>> 99 |
| rac-9b (s.Bsp.3) | 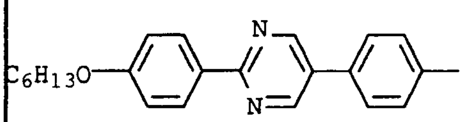 | Cr<br>$S_C$<br>N<br>is | < 59<br>59 - 95<br>95 - 97<br>> 97 |

**Patentansprüche**

1. Flüssigkristalline Allene der allgemeine Formel I

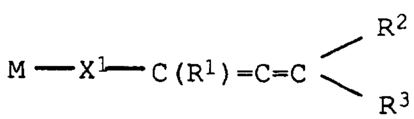

I

in der die Substituenten folgende Bedeutung haben:

| | |
|---|---|
| M | eine mesogene Gruppe |
| $R^1$, $R^2$ | Wasserstoff oder C-organische Reste mit 1 - 8 C-Atomen |
| $R^3$ | einen C-organischen Rest mit 1 - 30 C-Atomen |
| $X^1$ | $-(CH_2)_q-O-(CH_2)_p-$,<br>$-(CH_2)_q-NR^4-(CH_2)_p-$,<br>$-(CH_2)_q-CO-O-(CH_2)_p-$,<br>$-(CH_2)_q-O-CO-(CH_2)_p-$,<br>$-(CH_2)_q-CO-NR^4-(CH_2)_p-$<br>$-(CH_2)_q-NR^4-CO-(CH_2)_p-$ |
| $R^4$ | Wasserstoff oder einen Alkylrest mit 1-4 C-Atomen, |
| p | 1-20 und |
| q | 0-20, |

wobei die Definitionen für $R^2$ und $R^3$ auch vertauscht sein können.

2. Flüssigkristalline Allene nach Anspruch 1, in denen M ein Rest der allgemeinen Formel Ia ist

$$R^5-X^{1a}-Z-A^1-Z-A^2-Z-(A^3)_m-Z-(A^4)_n- \qquad \text{Ia}$$

wobei die Variablen folgende Bedeutung haben:

| | |
|---|---|
| $A^1$-$A^4$ | iso- oder heteroaromatische oder iso- oder heterocycloaliphatische Gruppen, die ihrerseits Substituenten tragen können |
| $R^5$ | einen C-organischen Rest mit 1-30 C-Atomen |
| $X^{1a}$ | eine Gruppe $X^1$ oder eine chemische Bindung |
| Z | gleiche oder verschiedene Brückenglieder der Struktur -CO-O-, -O-CO-, -CH=CH-, -N=N-, -CH=N-, -N=CH-, -CO-$NR^4$-, -$NR^4$-CO-, -$CH_2$-O-, -O-$CH_2$- oder eine chemische Bindung |
| m,n | 0 oder 1 |

**3.** Flüssigkristalline Allene nach Anspruch 2, in denen einer der Reste $A^1$-$A^4$ ein heteroaromatischer Rest ist und die übrigen Reste 1,4-Phenylengruppen bedeuten.

**4.** Flüssigkristalline Allene nach Anspruch 3, in denen die Gruppierung

$$-A^1-Z-A^2-Z-(A^3)_m-Z-(A^4)_n-$$

für die folgende Gruppe steht

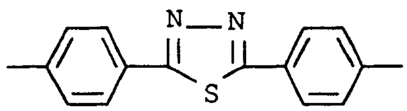

**5.** Allene der allgemeinen Formel IIa

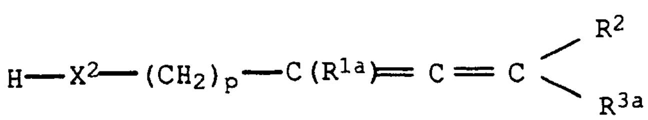

IIa,

in der die Variablen folgende Bedeutung haben

| | |
|---|---|
| p,$R^2$ | wie in Anspruch 1 genannt |
| $R^{1a}$ | einen C-organischen Rest mit 1-8 C-Atomen |
| $R^{3a}$ | einen C-organischen Rest mit 7-30 C-Atomen |
| $X^2$ | -0-; -$NR^4$-; -O-CO- |

wobei die Definitionen für $R^2$ und $R^{3a}$ auch vertauscht sein können.

6. Die Verbindungen

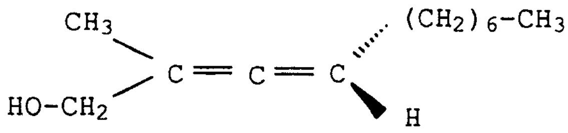

und

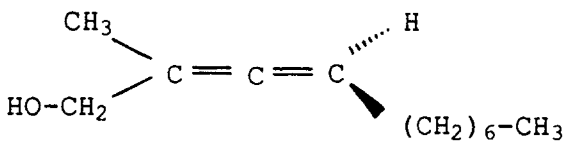

7. Verfahren zur Herstellung der flüssigkristallinen Allene gemäß den Ansprüchen 1 bis 4, dadurch gekennzeichnet, daß man ein Allen der allgemeinen Formel II

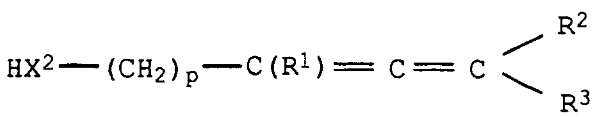

II

in der $R^1$, $R^2$, $R^3$ und p die Bedeutung wie in Anspruch 1 und $X^2$ die Bedeutung wie in Anspruch 5 haben, mit einer Verbindung III

$$M\text{-}X^3 \qquad \text{III}$$

in der $X^3$ für eine reaktive Gruppe steht, welche mit $HX^2$-unter Ausbildung einer Ether-, Amino-, Ester- oder Amidverknüpfung reagieren kann.

8. Verwendung der flüssigkristallinen Allene gemäß den Ansprüchen 1 bis 4 in optischen Anzeigeelementen, in elektrooptischen Speichermedien, in elektrophotographischen Geräten sowie in lichtreflektierenden Schichten.